# EUROPÄISCHE PATENTANMELDUNG

(11) **EP 3 520 844 A1**
(43) Veröffentlichungstag der Anmeldung: **07.08.2019**
(21) Anmeldenummer: 19173016.7
(22) Anmeldetag: 07.05.2019
(51) Int. Cl.: A61M 5/315

(54) **DOSIEREINRICHTUNG FÜR EINE INJEKTIONSVORRICHTUNG**

(71) Anmelder: Tecpharma Licensing AG, 3401 Burgdorf (CH)
(72) Erfinder: Hostettler, Patrick, 3415 Hasle (CH); Scheurer, Simon, 3006 Bern (CH); Hirschel, Jürg, 3007 Bern (CH)

(57) **Zusammenfassung**

Die Erfindung betrifft eine Injektionsvorrichtung (1) zum Ausschütten einer Dosis eines Produkts mittels manuellem Vorschub einer Kolbenstange (70), mit einem Dosier- und Ausschüttmechanismus. Dieser umfasst ein Gehäuse (10) mit einer Längsachse, eine im Gehäuse (10) gelagerte Dosierhülse (30) zum Einstellen und Korrigieren der Dosis, einen Gehäuseeinsatz (20), welcher gehäusefest ist und innerhalb der Dosierhülse (30) angeordnet ist, eine im Gehäuse (10) gelagerte Kupplungshülse (50) zum Antreiben der Kolbenstange (70). Zum Einstellen und Korrigieren einer Dosis ist die Dosierhülse (30) relativ zur Kupplungshülse (50) drehbar und zum Ausschütten der Dosis ist die Dosierhülse (30) mit der Kupplungshülse (50) rotationsfest koppelbar. Dabei weist die Kupplungshülse (50) ein Sperrelement auf, welches mit dem Gehäuseeinsatz (20) zusammenwirken kann, so dass die Kupplungshülse (50) in eine erste Drehrichtung rotierbar und in eine zweite Drehrichtung nicht rotierbar ist relativ zum Gehäuseeinsatz (20).

## Beschreibung

### TECHNISCHES GEBIET

Die vorliegende Erfindung betrifft das Gebiet der medizinischen Injektionsvorrichtungen zur Verabreichung von flüssigen Substanzen insbesondere von Medikamenten oder medizinischen Substanzen wie Insulin- und Hormonpräparationen. Die Erfindung bezieht sich auf eine Injektionsvorrichtung mit einem Dosier- und Ausschüttmechanismus.

### HINTERGRUND DER ERFINDUNG

Bekannte Injektionsgeräte umfassen üblicherweise eine Antriebshülse und/oder eine Kupplungshülse zum Antreiben einer Kolbenstange um die flüssigen Substanz aus einer Karpule oder einem Produktbehälter auszuschütten, ein Dosierelement zum Einstellen einer zu verabreichenden Dosis, ein Ausschüttknopf und einen Kupplungsmechanismus zum wahlweisen koppeln des Dosierelements mit der Antriebshülse oder Kupplungshülse.

Um eine Dosis einzustellen, dreht oder zieht der Benutzer am Dosierelement, welches sich dabei aus dem Gehäuse des Injektionsgeräts herausschraubt bzw. herausbewegt. Damit die so eingestellte Dosis ausgeschüttet werden kann, drückt der Benutzer an einem proximalen Ende des Injektionsgeräts auf den Ausschüttknopf und erzeugt dadurch eine Kraft in distaler Richtung, wodurch das Dosierelement in das Gehäuse eingeschraubt oder eingeschoben wird. Im Unterschied zum Einstellen einer Dosis überträgt beim Ausschütten das Dosierelement die Dreh- oder Schiebebewegung auf die Antriebshülse oder Kupplungshülse, welche dadurch die Kolbenstange antreibt um die Substanz aus der Karpule auszuschütten. Je nach Ausführung ist dabei die Kolbenstange drehbar bzw. schraubbar oder nur verschiebbar relativ zum Gehäuse gelagert.

Um eine versehentlich zu hoch eingestellte Dosis zu korrigieren, kann der Benutzer das Dosierelement in das Gehäuse zurückdrehen oder zurückschieben. Bei dieser Korrekturbewegung wie auch bei der Handhabung des Injektionsgeräts zwischen den Verabreichungen muss sichergestellt werden, dass sich die Position der Kolbenstange nicht ungewollt relativ zur Karpule verstellen kann, insbesondere dass sich die Kolbenstange nicht von der Karpule wegbewegt.

Eine Möglichkeit ein ungewolltes Verstellen zu verhindern, ist die Verwendung einer sogenannten Rückdrehsicherung. Diese verhindert entweder direkt das Rotieren der Kolbenstange entgegen der Rotationsrichtung zum Ausschütten der Substanz oder die Rückdrehsicherung greift am antreibenden Antriebselement an, so dass eine Rückbewegung der Kolbenstange indirekt verhindert wird.

Die Patentschrift EP 2 221 077 B1 beschreibt einen Injektor umfassend ein Gehäuse in dem sich ein gehäusefest angeordneter hülsenförmiger Gehäuseeinsatz befindet. Auf seiner Aussenseite umfasst dieser ein Gewinde, in welches eine Dosierhülse eingreift. Im Innern ist eine drehbare Kolbenstange angeordnet, welche zudem in Gewindeverbindung mit dem Gehäuse steht. Weiter umfasst der Injektor eine Kupplungshülse, welche sich zwischen der Kolbenstange und dem Gehäuseeinsatz befindet. Zum Einstellen einer Dosis wird die Dosierhülse relativ zum Gehäuse aus diesem herausgeschraubt. Die Kupplungshülse rotiert dabei nicht, da sie rotationsfest mit der Kolbenstange gekoppelt ist. Die Kolbenstange ist mittels Ratschenelementen gehalten. Die am Gehäuse befindlichen Ratschenelemente sind im Bereich angeordnet, wo die Kolbenstange in das Gehäuse eingeschraubt ist. Sie erlauben eine Rotationsbewegung der Kolbenstange relativ zum Gehäuse in eine Ausschüttrichtung und blockieren eine Rotation in eine Gegenrichtung. Zum Ausschütten wird mittels Betätigung eines Ausschüttknopfs die Dosierhülse mit der Kupplungshülse gekoppelt und die Dosierhülse und die Kupplungshülse werden in das Gehäuse zurückgeschraubt. Da die Kolbenstange rotationsfest mit der Kupplungshülse verbunden ist, wird diese in Ausschüttrichtung rotiert, wodurch das Produkt ausgeschüttet werden kann.

Die mit der Kolbenstange zusammenwirkenden Ratschenelemente sind dabei direkt am Gehäuse angeordnet und befinden sich in einem axialen Abschnitt, welcher distal an die Kupplungshülse anschliesst. Die Injektionsvorrichtung ist dadurch länger.

### DARSTELLUNG DER ERFINDUNG

Es ist eine Aufgabe der Erfindung eine kompakt konstruierbare Injektionsvorrichtung zu schaffen.

Diese Aufgabe wird gelöst durch eine Injektionsvorrichtung und einen Dosier- und Ausschüttmechanismus gemäss den unabhängigen Ansprüchen. Bevorzugte Ausführungsformen sind Gegenstand der abhängigen Ansprüche.

Gemäss der Erfindung umfasst eine Injektionsvorrichtung zum Ausschütten einer Dosis eines Produkts mittels manuellem Vorschub einer Kolbenstange in eine in der Injektionsvorrichtung gehaltenen Karpule einen Dosier- und Ausschüttmechanismus. Dieser umfasst ein Gehäuse mit einer Längsachse, eine im Gehäuse gelagerte Dosierhülse zum Einstellen und Korrigieren der Dosis, einen Gehäuseeinsatz, welcher gehäusefest und innerhalb der Dosierhülse angeordnet ist und eine im Gehäuse gelagerte Kupplungshülse zum Antreiben der Kolbenstange. Zum Einstellen und Korrigieren einer Dosis ist die Dosierhülse relativ zur Kupplungshülse drehbar und zum Ausschütten der Dosis ist die Dosierhülse mit der Kupplungshülse rotationsfest koppelbar. Dabei weist die Kupplungshülse ein Sperrelement auf, welches mit dem Gehäuseeinsatz so zusammenwirken kann, dass die Kupplungshülse in eine erste Drehrichtung rotierbar und in eine zweite Drehrichtung nicht rotierbar ist relativ zum Gehäuseeinsatz.

Indem einerseits die Kupplungshülse das Sperrelement umfasst und andererseits der Dosier- und Ausschüttmechanismus einen Gehäuseeinsatz aufweist mit dem das Sperrelement zusammenwirken kann, ist eine kompakte Konstruktion der Injektionsvorrichtung ermöglicht. So ermöglicht der innerhalb der Dosierhülse angeordnete Gehäuseeinsatz, dass das Sperrelement im Inneren der Injektionsvorrichtung an einem gehäusefesten Element gehalten werden kann bzw. mit diesem zusammenwirken kann. Dadurch kann das Sperrelement beispielsweise direkt auf einer Aussenseite der Kupplungshülse angeordnet werden oder in die Kupplungshülse integriert werden. Das Sperrelement muss also nicht proximal oder distal anschliessend an die Kupplungshülse oder in einem zusätzlichen Element vorgesehen werden, um eine ungewollte Rotation der Kolbenstange zu verhindern. Ferner muss ein an der Kupplungshülse angeordnetes Sperrelement auch nicht zwingend an einem distalen oder proximalen Ende der Kupplungshülse angeordnet werden, was zusätzlicher Platz benötigen würde, um mit dem Gehäuse zusammenwirken zu können. Hingegen kann das Sperrelement an einer beliebigen axialen Position der Kupplungshülse angeordnet werden, wodurch der Dosier- und Ausschüttmechanismus kompakter insbesondere kürzer konstruiert werden kann.

Die Injektionsvorrichtung umfasst eine Karpule, in der sich eine medizinische Substanz befindet. Um das Produkt aus der Karpule auszuschütten, kann mittels der Kolbenstange der Injektionsvorrichtung beispielsweise ein Stopfen in der Karpule in einer Ausschüttrichtung in distaler Richtung verschoben werden. Vorzugsweise steht diese Ausschüttrichtung parallel zur Richtung der Längsachse des Gehäuses.

Die Injektionsvorrichtung ist vorzugsweise als Einweg-Injektionsvorrichtung ausgeführt, welche, nachdem der Inhalt der Karpule ausgeschüttet ist, entsorgt bzw. rezykliert wird. In einer alternativen Ausführung besteht aber auch die Möglichkeit, dass die Injektionsvorrichtung als Mehrweg-Injektionsvorrichtung ausgebildet ist. In diesem Fall ist durch eine lösbare Verbindung eines Karpulenhalters mit dem Gehäuse oder durch ein mehrteiliges Gehäuse die Karpule aus der Injektionsvorrichtung entnehmbar und eine neue Karpule einsetzbar.

Der Dosier- und Ausschüttmechanismus umfasst ein Gehäuse, in welchem die Dosierhülse, die Kupplungshülse und die Kolbenstange angeordnet sind. Dabei kann dieses Gehäuse gleichzeitig ein äusseres Gehäuse der Injektionsvorrichtung bilden oder die Injektionsvorrichtung kann alternativ eine zusätzliche Gehäusestruktur umfassen. In diesem Fall ist das Gehäuse des Dosier- und Ausschüttmechanismus vorzugsweise innerhalb der äusseren Gehäusestruktur der Injektionsvorrichtung angeordnet.

Das Gehäuse umfasst einen Gehäuseeinsatz, welcher beispielsweise die Form einer Hülse oder einer Halbschale aufweist und vorzugsweise koaxial zur zentralen Längsachse des Gehäuses angeordnet ist. Der Gehäuseeinsatz lagert vorzugsweise die Dosierhülse, insbesondere mittels Gewindeverbindung. Weiter kann der Gehäuseeinsatz weitere Elemente wie zum Beispiel die Kupplungshülse führen oder lagern.

Die Dosierhülse dient zum Einstellen und Korrigieren einer Dosis. Hierzu kann die Dosierhülse drehbar oder drehbar und verschiebbar relativ zum Gehäuse gelagert sein. Vorzugsweise ist die Dosierhülse mittels einer Schraubbewegung relativ zum Gehäuse bewegbar. Die Dosierhülse kann dabei beispielsweise ringförmig oder hülsenförmig ausgebildet sein. In einer bevorzugten Ausführung ist die Dosierhülse koaxial zur Längsachse des Gehäuses im Gehäuse angeordnet. Dabei umschliesst die Dosierhülse vorzugsweise den Gehäuseeinsatz, so dass die Dosierhülse radial gesehen zwischen einer Innenseite des äusseren Gehäuses und der Aussenseite des Gehäuseeinsatzes in einem Ringspalt angeordnet ist.

Die Kupplungshülse dient dazu beim Ausschütten der Dosis die Dosierhülse mit der Kolbenstange zu koppeln, insbesondere rotativ zu koppeln. Zudem kann die Kupplungshülse direkt oder indirekt über ein weiteres Element wie beispielsweise eine Antriebshülse die Kolbenstange antreiben. Vorzugsweise kann die Kupplungshülse direkt mit der Kolbenstange zusammenwirken, um diese anzutreiben. Die angetriebene Kolbenstange wird vorzugsweise in die Karpule hineinbewegt, so dass das Produkt aus der Karpule ausgeschüttet werden kann. Die Kolbenstange kann dabei durch die Kupplungshülse oder ein weiteres Element gedreht werden, so dass sie sich beispielsweise durch eine Schraubbewegung in distaler Richtung bewegt. Alternativ kann die Kolbenstange durch die Kupplungshülse oder ein weiteres Element nur verschoben werden und rotationsfest im Gehäuse geführt sein.

Beim Einstellen und Korrigieren einer Dosis wird die Kupplungshülse bevorzugt nicht rotiert, sondern nur axial relativ zum Gehäuse verschoben. Um eine eingestellte Dosis auszuschütten, wird durch eine manuell durch den Benutzer aufgebracht Kraft die Kupplungshülse und die mit ihr gekoppelte Dosierhülse in das Gehäuse zurückbewegt, insbesondere mittels einer Schraubbewegung in das Gehäuse geschraubt. Die Kraft kann entweder direkt vom Benutzer auf die Kupplungshülse ausgeübt werden oder indirekt über ein weiteres Element wie beispielsweise einen Ausschüttknopf. Dabei bedeutet "manuell", dass keine sonstige Energie wie beispielsweise eine Kraft aus einer vorgespannten Feder zum Ausschütten verwendet wird. Der Vorschub der Kolbenstange zum Ausschütten des Produkts wird also nur durch den Benutzer aufgebracht.

Das Sperrelement kann mit dem Gehäuseeinsatz zusammenwirken, so dass die Kupplungshülse relativ zum Gehäuseeinsatz in eine erste Richtung rotieren kann und in eine zweite, der ersten Richtung entgegengesetzten Richtung nicht rotieren kann bzw. blockiert ist. Das Sperrelement kann beispielsweise als Arm, Nocken, Zahn, Ratsche oder als sonstige Auskragung ausgebildet sein, welche mit dem Gehäuseeinsatz zusammen wirken kann. Das Sperrelement ist jedoch vorzugsweise nicht ein separates Bauteil, sondern ist einteilig oder mehrteilig mit der Kupplungshülse verbunden bzw. einstückig mit dieser.

In der vorliegenden Beschreibung bezeichnet der Begriff "distal" eine zum vorderen, einstechseitigen Ende der Injektionsvorrichtung beziehungsweise zur Spitze der Injektionsnadel hin gerichtete Seite oder Richtung. Demgegenüber bezeichnet die Angabe "proximal" eine zum hinteren, dem einstechseitigen Ende gegenüberliegenden Ende der Injektionsvorrichtung hin gerichtete Seite oder Richtung.

Der Begriff axial bezieht sich auf die Längsachse des Gehäuses. Entsprechend ist eine axiale Richtung parallel zur Längsachse des Gehäuses oder in Längsrichtung des Gehäuses. Eine radiale Richtung bezieht sich auf eine Richtung rechtwinklig zur Längsachse des Gehäuses.

Der Begriff "Produkt", "Medikament" oder "medizinische Substanz" umfasst im vorliegenden Zusammenhang jede fliessfähige medizinische Formulierung, welche geeignet ist zur kontrollierten Verabreichung mittels einer Kanüle oder Hohlnadel in subkutanes oder intramuskuläres Gewebe, beispielsweise eine Flüssigkeit, eine Lösung, ein Gel oder eine feine Suspension enthaltend einen oder mehrere medizinische Wirkstoffe. Ein Medikament kann also eine Zusammensetzung mit einem einzigen Wirkstoff oder eine vorgemischte oder co-formulierte Zusammensetzung mit mehreren Wirkstoffen aus einem einzelnen Behälter sein. Der Begriff umfasst insbesondere Arzneien wie Peptide (z.B. Insuline, Insulin enthaltende Medikamente, GLP 1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen sowohl in fester (suspendierter) oder flüssiger Form. Der Begriff umfasst weiter auch Polysaccharide, Vakzine, DNS oder RNS oder Oligonukleotide, Antikörper oder Teile von Antikörpern sowie geeignete Basis-, Hilfs- und Trägerstoffe.

Unter den Begriffen "Injektionsvorrichtung" oder "Injektor" wird in der vorliegenden Beschreibung eine Vorrichtung verstanden, bei der die Injektionsnadel nach erfolgter Abgabe einer kontrollierten Menge der medizinischen Substanz aus dem Gewebe entfernt wird. Somit verbleibt bei einem Injektionssystem oder bei einem Injektor im Unterschied zu einem Infusionssystem die Injektionsnadel nicht über einen längeren Zeitraum von mehreren Stunden im Gewebe.

Unter "Einweg-Injektionsvorrichtung", welche auch als "disposable" bezeichnet wird, ist eine Vorrichtung zu verstehen, welche nach einem oder mehreren Injektionsvorgängen jedoch spätestens nach der letzten ausschüttbaren Dosis entsorgt wird. Die Karpule ist bei einer solchen Einweg-Injektionsvorrichtung nicht auswechselbar. Demgegenüber kann bei Mehrweg-Injektionsvorrichtungen oder "reusable" Injektionsvorrichtungen die eingesetzte Karpule ausgetauscht werden, wenn das Produkt vollständig aus dieser ausgeschüttet worden ist. Bei solchen Mehrweg-Injektionsvorrichtungen wird beim Austausch der Karpule die Kolbenstange wieder in proximaler Richtung zurück ins Gehäuse geschoben, so dass diese wieder bereit zum Ausschütten des Produkts aus der neue Karpule ist.

Bevorzugt ist das Sperrelement ein elastischer radialer Arm. Dabei kann mindestens ein Bereich des Arms in ein Gegenelement im Innern des Gehäuseeinsatzes eingreifen, um so eine Rotation der Kupplungshülse in eine Richtung zu ermöglichen und in die entgegengesetzte Richtung zu blockieren. Dadurch ist ein einfacher und kompakter Mechanismus realisierbar, welcher die Drehrichtung der Kupplungshülse kontrollieren kann. Der Arm ist vorzugsweise an einem ersten Ende unbeweglich mit der Kupplungshülse verbunden. Das zweite, freie Ende des Arms kann hingegen mit dem Gehäuseeinsatz zusammenwirken. Der Arm weist hierzu vorzugsweise an seinem freien Ende ein Eingriffsbereich auf. Vorzugsweise ist der Arm in Bezug auf die axiale Länge der Kupplungshülse in einem mittleren Bereich der Kupplungshülse angeordnet.

Alternativ dazu besteht auch die Möglichkeit, dass das Sperrelement nicht als Arm sondern beispielsweise als Nocken, Zahn oder Ratschenelement ausgebildet ist.

Die Hülsenform der Kupplungshülse wird durch eine Mantelfläche gebildet. In einer bevorzugten Ausführung ist der Arm in dieser Mantelfläche der Kupplungshülse ausgebildet. Der Arm ist bevorzugt integriert in diese Mantelfläche, so dass der Arm mit einem Ende mit der Kupplungshülse verbunden ist und mit seinem anderen, freien Ende relativ zur Mantelfläche elastisch und flexibel bewegbar ist. Dadurch kann der Arm besonders platzsparend konstruiert werden, da er nicht an einem distalen oder proximalen Ende der Kupplungshülse platziert werden muss, sondern platzsparend in deren Mantelfläche integriert werden kann.

In einer bevorzugten Ausführung ist der Arm einteilig mit der Kupplungshülse ausgebildet. Dadurch kann die Anzahl der Bauteile reduziert werden, was die Montage vereinfacht.

Bevorzugt umfasst der Gehäuseeinsatz in Richtung Längsachse ausgerichtete Nuten, mit denen das Sperrelement zusammenwirken kann. Die Nuten sind vorzugsweise im Innern des Gehäuseeinsatzes ausgebildet, insbesondere auf einer Innenfläche einer hülsen- oder schalenförmigen Gestalt des Gehäuseeinsatzes.

Dabei sind die Nuten und /oder ein Teil des Sperrelements vorzugsweise im Querschnitt asymmetrisch ausgestaltet, so dass das Sperrelement in Form eines Arms, Nockens oder einer Auskragung in einer Richtung über die Nuten bewegt werden kann und in der Gegenrichtung in die Nuten eingreift und so eine Relativbewegung zwischen Kupplungshülse und Gehäuseeinsatz blockieren kann. So können die Nuten beispielsweise derart geformt sein, dass zwischen den Nuten im Querschnitt sägezahnförmige Elemente bestehen, welche das Sperrelement in einer Richtung passieren lassen und in der Gegenrichtung blockieren.

Alternativ dazu besteht auch die Möglichkeit, der Gehäuseeinsatz keine Nuten aufweist sondern ein Gegenelement wie beispielsweise einzelne asymmetrisch geformte Zähne oder Auskragungen, welche mit dem Sperrelement zusammenwirken können.

Bevorzugt weist der Gehäuseeinsatz auf seiner Aussenseiten ein Gewinde auf, mit dem die Dosierhülse in Gewindeeingriff steht. Die Dosierhülse ist in diesem Fall radial zwischen einer Inneren Wandung einer äusseren Gehäusehülse und einer Aussenseite des Gehäuseeinsatzes platziert. Dadurch kann die Dosierhülse platzsparend in der Injektionsvorrichtung angeordnet werden. Da die Dosierhülse das Gewinde auf ihrer Innenseite aufweist, ist auf der Aussenseite kein Gewinde vonnöten, wodurch Anzeigeelemente wie beispielsweise eine Zahlenskala gut ablesbar angeordnet werden können.

Vorzugsweise weist die Kupplungshülse ein elastisches Klickelement auf, welches beim Einstellen und Korrigieren einer Dosis in der ersten wie auch in der zweiten Drehrichtung mit der Dosierhülse zusammenwirken kann zum Erzeugen eines akustischen und/oder taktilen Signals. Das Klickelement kann somit für beide Drehrichtungen ein Signal erzeugen und es muss nicht, wie im Stand der Technik üblich, je ein Element pro Drehrichtung verwendet werden. Dies wird zum Beispiel durch die Form des Klickelements mit einem abgerundeten freien Ende ermöglicht. So kann das Klickelement beispielsweise ein elastischer Arm, Zahn oder Schieber sein. Bevorzugt wird das Klickelement über Nocken, Nuten oder Zähne der Dosierhülse bewegt, wodurch das akustische und/oder taktile Signal zum Beispiel in Form eines Klicks und/oder einer Vibration erzeugt werden kann.

Ferner ist bevorzugt die Kupplungshülse rotationsfest jedoch verschiebbar relativ zur Kolbenstange gelagert und zudem direkt an der Kolbenstange gelagert. Dadurch kann die Kupplungshülse beim Einstellen und Korrigieren einer Dosis relativ zur Kolbenstange verschoben werden. Falls die Kolbenstange drehbar gelagert ist relativ zum Gehäuse, kann diese beim Ausschütten durch die rotativ verbundene Kupplungshülse rotiert werden. Vorzugsweise steht dabei die Kolbenstange in Gewindeeingriff mit dem Gehäuse, so dass sich beim Ausschütten die Kolbenstange nach distal durch das Gehäuse schraubt.

Dabei weist die Kupplungshülse vorzugsweise auf ihrer Innenseite mindestens einen zum Zentrum hin weisenden axialen Steg auf. Das bedeutet, der Steg ist auf der Innenseite der Kupplungshülse angeordnet, wobei die Höhe des Stegs zum Rotationszentrum der Kupplungshülse hinweist. Die Kolbenstange umfasst vorzugsweise eine entsprechende Nut, in welche der Steg eingreifen kann. Falls die Kolbenstange ein Aussengewinde aufweist, ist dieses durch die Nut unterbrochen. Mittels der Verbindung mit dem Steg und der Nut ist die Kupplungshülse sicher und zuverlässig rotativ mit der Kolbenstange gekoppelt aber dennoch in Richtung Längsachse relativ zur Kolbenstange verschiebbar gelagert.

In einer weiteren bevorzugten Ausführung ist die Kupplungshülse zweiteilig ausgebildet. Sie umfasst in diesem Fall einen ersten Teil, welcher mit der Kolbenstange zusammenwirken kann und einen zweiten Teil, welcher mit der Dosierhülse koppelbar ist, wobei der erste und der zweite Teil relativ zueinander verschiebbar und rotationsfest gelagert sind bzw. miteinander verbunden sind.

Dabei ist der erste Teil vorzugsweise axialfest aber rotierbar am Gehäuse oder am Gehäuseeinsatz gelagert. Der zweite Teil ist in Richtung Längsachse relativ zum ersten Teil verschiebbar. Dadurch kann beim Einstellen und Korrigieren einer Dosis, wenn die Dosierhülse relativ zum Gehäuse bewegt wird, der zweite Teil der Kupplungshülse und die Dosierhülse zusammen aus dem Gehäuse heraus bewegt werden. Der zweite Teil bleibt dabei rotativ fest mit dem ersten Teil gekoppelt, welcher sich in Längsrichtung nicht bewegt. Dadurch ist sichergestellt, dass der erste Teil stets mit der Kolbenstange zusammenwirken kann. Das bedeutet, die Kolbenstange muss nicht besonders lang ausgebildet werden, da der erste Teil axial nicht bewegt wird. Bei einer einteiligen Kolbenstange besteht die Gefahr, dass die Kupplungshülse beim Einstellen einer Dosis von der Kolbenstange wegbewegt wird, so dass sie die Kolbenstange nicht mehr antreiben kann.

Bevorzugt befindet sich das Sperrelement am zweiten Teil der Kupplungshülse, so dass dieses mit dem Gehäuseeinsatz zusammenwirken kann. Fall die Kupplungshülse ein elastisches Klickelement aufweist zum Erzeugen eines akustischen und/oder taktilen Signals ist dieses vorzugsweise auf einer Aussenseite des zweiten Teils der Kupplungshülse angeordnet, so dass das elastische Klickelement mit der Dosierhülse zusammenwirken kann.

Dabei ist vorzugsweise der erste Teil verschiebbar und rotationsfest mit der Kolbenstange relativ zur Kolbenstange an dieser gelagert. Ferner ist der erste Teil bevorzugt hülsenförmig ausgebildet und koaxial im zweiten Teil angeordnet, welcher ebenfalls hülsenförmig ausgebildet ist, so dass der zweite Teil den ersten Teil umgibt. Eine solche Anordnung ermöglicht eine platzsparende Konstruktion.

Falls die Kupplungshülse auf ihrer Innenseite einen axialen Steg aufweist, ist dieser vorzugsweise im Innern des ersten hülsenförmigen Teils ausgebildet, so dass der Steg mit einer allenfalls vorhandenen axialen Nut in der Kolbenstange eingreifen kann.

### FIGUREN

In Zusammenhang mit den angehängten Figuren werden nachfolgend bevorzugte Ausführungsformen der Erfindung beschrieben. Diese sollen grundsätzliche Möglichkeiten der Erfindung aufzeigen und keinesfalls einschränkend ausgelegt werden.
- Fig. 1: zeigt in perspektivischer Ansicht eine Explosionsdarstellung des erfindungsgemässen Injektors mit einem Dosier- und Ausschüttmechanismus;
- Fig. 2: zeigt den Injektor in einer Schnittansicht in einer Ausgangsstellung, wobei der Schnitt durch die Längsachse des Injektors verläuft;
- Fig. 3: zeigt die Schnittansicht des Injektors nachdem eine Dosis eingestellt wurde;
- Fig. 4: zeigt eine Schnittansicht durch den mittleren Bereich des Injektors, wobei der Schnitt rechtwinklig zur Längsachse des Injektors verläuft;
- Fig. 5: zeigt eine Schnittansicht in einem proximalen Bereich des Injektors, wobei der Schnitt rechtwinklig zur Längsachse verläuft;
- Fig. 6: zeigt eine zweite Ausführung des Injektors in einer Schnittansicht in einer Ausgangsstellung;
- Fig. 7: zeigt die zweite Ausführung des Injektors nachdem eine Dosis eingestellt wurde.

### FIGURENBESCHREIBUNG

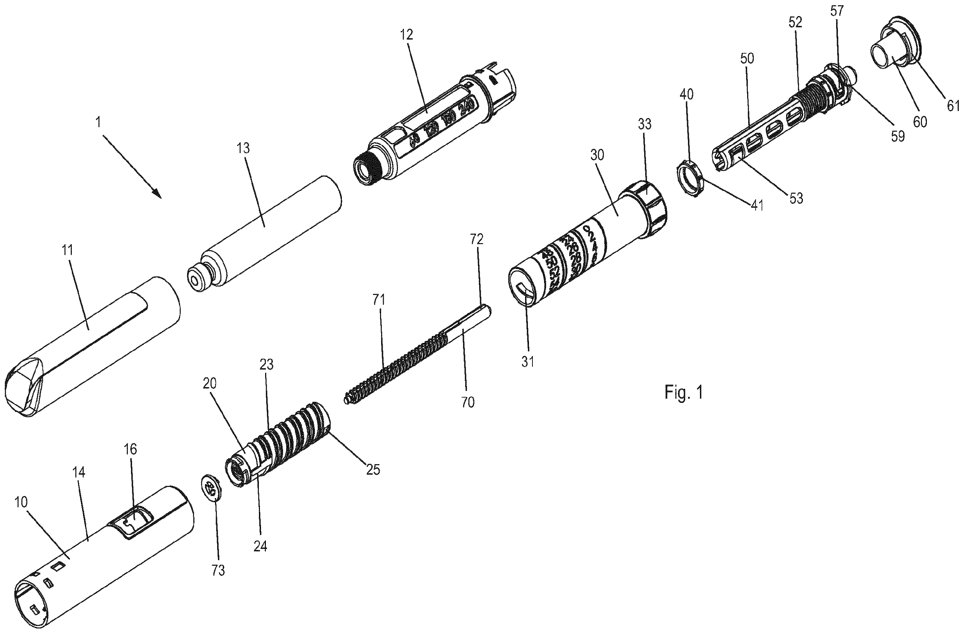

Die Figur 1 zeigt eine erfindungsgemässe Injektionsvorrichtung mit einem Dosier- und Ausschüttmechanismus in einer Explosionsdarstellung. Dabei befindet sich das distale, einstechseitige Ende des Injektors 1 im linken, unteren Bereich der Figur 1 und das proximale Ende des Injektors 1 im rechten, oberen Bereich der Figur 1.

Der Injektor 1 ist in der gezeigten Ausführung als Einweginjektor ausgebildet. Wie in Figur 1 ersichtlich, umfasst der Injektor 1 eine abnehmbare Schutzkappe 11, ein längliches, zylinderförmiges Gehäuse 10, welches zugleich das Gehäuse des Dosier- und Ausschüttmechanismus bildet und ein Karpulenhalter 12 in dem eine Karpule 13 mit einer medizinischen Substanz gehalten ist. Weiter umfasst der Injektor 1 den Dosier- und Ausschüttmechanismus. Dieser beinhaltet das Gehäuse 10, welches eine äussere Gehäusehülse 14 und eine im Innern der äusseren Gehäusehülse 14 angeordneter Gehäuseeinsatz in der Form einer innere Gehäusehülse 20 umfasst, eine Dosierhülse 30 zum Einstellen und Korrigieren einer Dosis, eine Kupplungshülse 50, eine Stoppmutter 40, welche in Gewindeverbindung mit der Kupplungshülse 50 steht, ein Ausschüttknopf 60 zum Auslösen des Ausschüttvorgangs und eine innerhalb der Kupplungshülse 50 angeordnete Kolbenstange 70, welche durch die Kupplungshülse 50 angetrieben werden kann zum Ausschütten der medizinischen Substanz aus der Karpule 13.

Nachfolgend wird auf die strukturellen Merkmale der einzelnen Bestandteile des Injektors 1 im Detail eingegangen. Die Funktion, insbesondere das Einstellen, Korrigieren und Ausschütten einer Dosis, ist im Anschluss beschrieben.

Der Karpulenhalter 12 ist an einem distalen Ende des Gehäuses 10 mittels einer Schnappverbindung rotationsfest und axialfest mit dem Gehäuse 10 verschnappt. Der Karpulenhalter 12 lagert die Karpule 13 und weist an seinem distalen Ende ein Verbindungselement auf, an dem eine Injektionsnadel (nicht dargestellt) angebracht werden kann.

Die äussere Gehäusehülse 14 ist zylinderförmig ausgestaltet. In einem proximalen Bereich ist ein radialer Durchbruch oder eine Öffnung 16 im Mantel der Gehäusehülse 14 ausgeformt. Durch diese Öffnung 16 ist von aussen eine Zahlenskala der Dosierhülse 30 ablesbar.

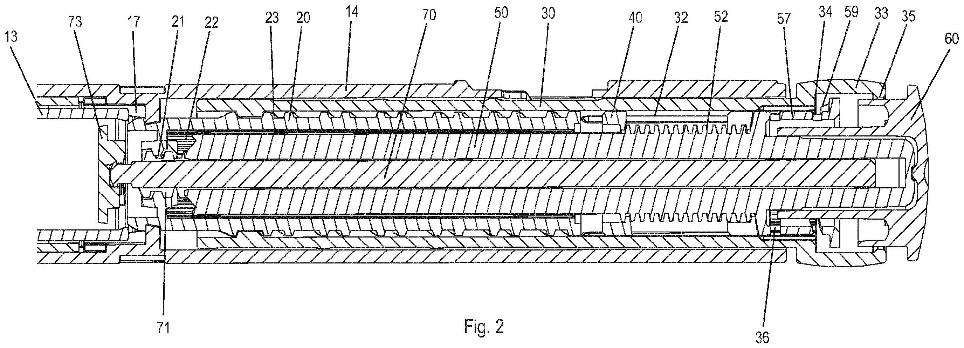

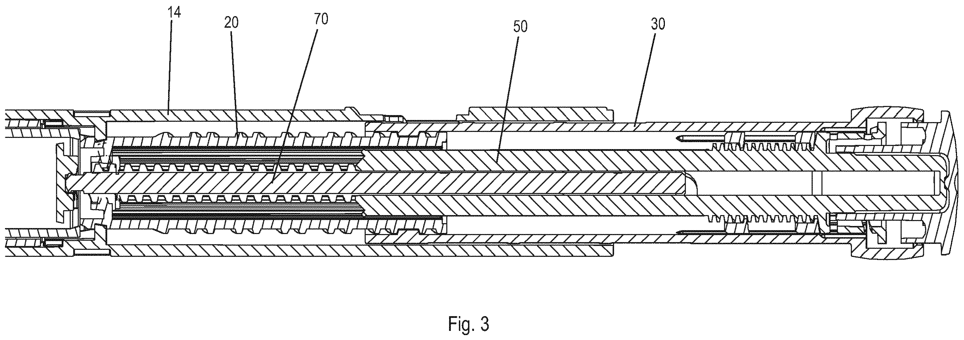

Ferner weist die äussere Gehäusehülse 14 am distalen Ende auf der Innenseite über den Umfang verteilt radial zum Zentrum hin ragende Rippen 17 auf, erkennbar in den Figuren 2 und 3. Die Rippen 17 weisen in axialer Richtung gesehen eine Steigung auf, wobei die Steigung in proximaler Richtung zunimmt. Mit anderen Worten ausgedrückt, sind die Rippen 17 axial keilförmig ausgebildet und weisen distal eine kleinere radiale Höhe auf als proximal, wobei die radiale Höhe stetig Richtung proximal zunimmt (Steigung). Die Rippen 17 sind dabei so dimensioniert bzw. materialisiert, dass sie plastisch verformbar sind. Die Karpule 13 wird zuerst in den Karpulenhalter 12 eingeführt. Anschliessend wird der Karpulenhalter 12 mit der äusseren Gehäusehülse 14 mittels einer Schnappverbindung verbunden. Beim Zusammenführen des Karpulenhalters 12 mit der Gehäusehülse 14 berührt zuerst die im Karpulenhalter 12 befindliche Karpule 13 bzw. deren Flammrand die Rippen 17 an einer zum Zentrum weisenden Kante. Wird der Karpulenhalter 12 weiter in proximale Richtung in die Gehäusehülse 14 hineingeschoben, deformieren sich die Rippen 17 plastisch. Die Rippen 17 werden also durch die Karpule 13 bleibend verformt. Dabei werden die Rippen 17 entweder als Ganzes aus ihrer ursprünglichen Position zur Seite gedrückt oder die in radialer Richtung verformt und nehmen zumindest teilweise die Form der Aussenkontur der Karpule 13 an. Wenn der Karpulenhalter 12 nun mit der Gehäusehülse 14 verschnappt wird, ist die Karpule 13 axial wie auch radial spielfrei und unbeweglich gehalten, da der Karpulenhalter 12 eine Spannkraft in proximaler Richtung auf die Karpule 12 ausübt und diese dadurch auf die verformten Rippen 17 drückt.

Die innere Gehäusehülse 20 hat eine zylindrische Form und ist koaxial zur äusseren Gehäusehülse 14 angeordnet. Mittels Schnapper, welche in eine Wandung auf der Innenseite der äusseren Gehäusehülse 14 eingreifen ist die innere Gehäusehülse 20 sowohl in axialer Richtung wie auch rotativ unbeweglich relativ zur äusseren Gehäusehülse 14 an dieser verschnappt. In einem distalen Bereich insbesondere durch einen inneren Boden weist die innere Gehäusehülse 20 ein Innengewinde 21 auf, in welchem die Kolbenstange 70 eingeschraubt ist, ersichtlich in den Figur 2 und 3. Ferner weist die innere Gehäusehülse 20 mehrere in Richtung Längsachse ausgerichtete Längsnuten 22 auf.

Auf ihrer Aussenseite weist die innere Gehäusehülse 20 ein Aussengewinde 23 auf, auf welches die Dosierhülse 30 geschraubt ist. Am distalen Ende und am proximalen Ende des Aussengewindes 23 ist je ein axial ausgerichteter Steg oder Absatz 24, 25 ausgebildet. In der vollständig eingeschraubten Position der Dosierhülse 30 schlägt ein Anschlag (nicht gezeigt) in der Dosierhülse am distalen Steg 24 an, so dass die minimale Dosis bzw. die Schraubbewegung der Dosierhülse 30 in das Gehäuse hinein durch den distalen Steg 24 begrenzt wird. Die maximale Dosis bzw. die Schraubbewegung der Dosierhülse 30 aus dem Gehäuse 10 heraus wird durch den proximalen Steg 25 begrenzt. Die innere Gehäusehülse 20 lagert somit die Dosierhülse 30 drehbar relativ zum Gehäuse 10.

Die Dosierhülse 30 hat die Form eines Hohlzylinders oder einer Hülse und weist am proximalen Abschluss einen Bereich mit einem Durchmesser auf, welcher grösser ist als der übrige Bereich der Dosierhülse 30 und welcher als Griff 33 dient zum Drehen der Dosierhülse 30. Dieser Griff 33 passt nicht in das Gehäuse 10, sondern steht, wie in Figur 2 ersichtlich, am proximalen Abschluss der äusseren Gehäusehülse 14 an. Am proximalen Ende des Griffs 33 weist dieser einen nach innen weisende umlaufenden Wulst 35 auf, ebenfalls erkennbar in Figur 2. Zudem ist auf der Aussenseite der Dosierhülse 30 eine Zahlenskala vorhanden, welche dem Benutzer die einstellbaren Dosen anzeigt.

Die Dosierhülse 30 steht, wie erwähnt, in Gewindeverbindung mit der inneren Gehäusehülse 20. Hierzu weist die Dosierhülse 30 auf ihrer Innenseite in einem distalen Bereich ein Innengewinde 31 auf. In einem mittleren Bereich weist die Dosierhülse 30 auf ihrer Innenseite mehrere axiale Nuten 32 für die Führung der Stoppmutter 40 auf. Weiter sind proximal an diese Nuten 32 anschliessend umlaufend radiale Zähne 36 ausgebildet, welche, wie unten beschrieben, mit Klickarmen 57 der Kupplungshülse 50 zusammenwirken können.

Beim Übergang vom Bereich des kleineren Durchmessers zum Bereich des grösseren Durchmessers besteht im Innern der Dosierhülse 30 eine ringförmige Fläche, welche rechtwinklig zur Längsachse steht. Auf dieser Fläche sind umlaufen axiale Zähne 34 angeordnet, die mittels eines abgerundeten Übergangs miteinander verbunden sind.

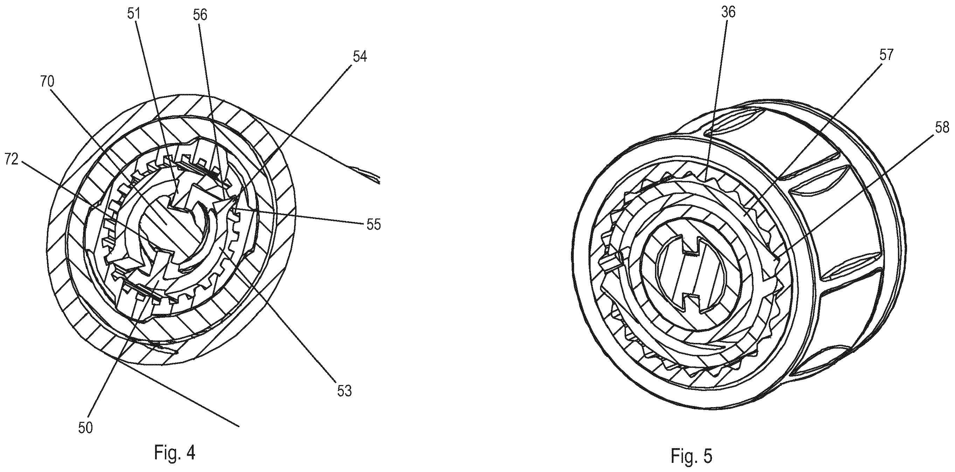

Die hohlzylinderförmige, längliche Kupplungshülse 50 weist von distaler Seite her eine Bohrung auf. In dieser befinden sich zwei in Umfangrichtung um 180° versetzte zum Zentrum hin ragende Stege 51, welche sich über die ganze axiale Länge der Bohrung erstrecken. Die Stege 51 sind in Figur 4 erkennbar.

Zudem weist die Kupplungshülse 50 einen distalen Abschnitt und einen proximalen Abschnitt auf, wobei zwischen dem distalen und proximalen Abschnitt ein hülsenförmiger Abschnitt angeordnet ist. Der distale Abschnitt weist in seinem proximalen Endbereich ein Aussengewinde 52 auf, in welches die Stoppmutter 40 eingreift. Zudem sind im distalen Abschnitt in der Mantelfläche flexible, elastische Ratschenarme 53 ausgebildet, welche als Sperrelement dienen. Die Ratschenarme 53 sind in der Mantelfläche der Kupplungshülse 50 integriert und haben dadurch eine bogenförmige Gestalt.

Wie in Figur 4 ersichtlich, weisen die Ratschenarme 53 an einem freien Ende je einen radial auskragenden Nocken 54 auf, welcher mit den Längsnuten 22 der inneren Gehäusehülse 20 zusammenwirken kann. Im montierten Zustand sind die elastischen Ratschenarme 53 leicht radial nach innen vorgespannt. Dadurch werden sie in die Längsnuten 22 gedrückt. Die Nocken 54 weisen auf einer Seite eine rechtwinklig zur Längsachse stehende Abschlussfläche 56 und auf der anderen Seite einen abgerundeten Bereich 55 auf, welcher einen fliessenden Übergang vom Nocken 54 zum bogenförmigen Ratschenarm 53 schafft.

Wenn nun die Kupplungshülse 50 relativ zur inneren Gehäusehülse 20 in eine Ausschüttrichtung rotiert wird, können die Ratschenarme 53 je mit dem abgerundeten Bereich 55 über die Längsnuten 22 gleiten. Das bedeutet, in dieser Drehrichtung greift der Nocken 54 nur kraftschlüssig in die Längsnuten 22 ein und kann bei Rotation aufgrund des abgerundeten Bereichs 55 von einer Längsnut 22 zur nächsten gleiten.

Wird hingegen die Kupplungshülse 50 in eine Gegenrichtung zur Ausschüttrichtung relativ zur Gehäusehülse 20 rotiert, greifen die Nocken 54 je in die Längsnut 22 ein. Bei dieser Drehrichtung stösst die gerade Abschlussfläche 56 des Nockes 54 an eine Nutflanke der Längsnut 22 an. Bedingt durch die Bogenform des Ratschenarms 53 wird bei einer weiteren Rotation der Nocken 54 tiefer bzw. formschlüssig in die Längsnuten 22 gedrückt, wodurch eine Rotation der Kupplungshülse 50 relativ zur Gehäusehülse 20 blockiert wird. Die Ratschenarme 53 ermöglichen somit eine Rotation der Kupplungshülse 50 relativ zur inneren Gehäusehülse 20 bzw. zum Gehäuse 10 in der Ausschüttrichtung und verhindern eine Rotation in die entgegengesetzte Drehrichtung.

Der hülsenförmige Abschnitt der Kupplungshülse 50 ist axial- und rotationsfest mit der Kupplungshülse 50 verbunden. Im hülsenförmigen Abschnitt sind in der Mantelfläche radiale Klickarme 57 ausgebildet. Wie in Figur 5 ersichtlich, weisen diese je am freien Ende einen abgerundeten Zahn 58 auf, welcher mit den radialen Zähnen 36 der Dosierhülse 30 zusammenwirkt. Weiter weist der hülsenförmige Abschnitt am proximalen Abschluss einen radial nach aussen weisenden Kragen auf, der eine ringförmige Fläche bildet, welche rechtwinklig zur Längsachse ausgerichtet ist. Auf dieser Fläche sind umlaufend axiale Zähne 59 angeordnet, welche mit den axialen Zähne 34 der Dosierhülse in Eingriff gebracht werden können.

Der proximale Abschnitt der Kupplungshülse 50 hat einen kleineren Durchmesser als der distale Abschnitt und dient dazu den Ausschüttknopf 60 drehbar zu lagern. Der Ausschüttknopf 60 ist mit einem kegelförmigen Lager im Zentrum am proximalen Ende der Kupplungshülse 50 auf dieser abgestützt. Zudem greift der Ausschüttknopf mit radialen Stegen 61 (Figur 1) hinter den Wulst 35 der Dosierhülse 30 ein, wodurch der Ausschüttknopf 60 in Richtung Längsachse relativ zur Dosierhülse 30 und zur Kupplungshülse 50 gehalten ist.

Wie erwähnt, ist auf dem Aussengewinde 52 der Kupplungshülse 50 die Stoppmutter 40 aufgeschraubt. Diese hat auf ihrer Aussenseite axial ausgerichtete Stege 41, welche in die Nuten 32 der Dosierhülse 30 eingreifen. Die Stoppmutter 40 ist demnach drehbar relativ zur Kupplungshülse 50 und axial verschiebbar aber rotationsfest relativ zur Dosierhülse 30.

Die Kolbenstange 70 umfasst ein Aussengewinde 71, mit dem sie in das Innengewinde 21 des inneren Gehäuseeinsatzes 20 eingeschraubt ist. Wie am besten in der Figur 4 erkennbar, weist die Kolbenstange 70 zwei in Umfangrichtung um 180° versetzte Nuten 72 auf, welche sich über die gesamte axiale Länge der Kolbenstange 70 erstrecken. In diese Nuten greifen im montierten Zustand die Stege 51 der Kupplungshülse 50 ein, wodurch die Kupplungshülse 50 rotativ aber verschiebbar mit der Kolbenstange 70 gekoppelt ist.

Am distalen Ende der Kolbenstange 70 befindet sich ein knopfförmiger Abschluss der Kolbenstange 70 (Figur 2), der eine Schnappverbindung mit einem Flansch 73 ermöglicht, wodurch der Flansch 73 relativ zur Kolbenstange 70 drehbar ist, jedoch in axialer Richtung an der Kolbenstange 70 unverschiebbar gehalten ist. Der Flansch 73 kann auf einen Stopfen in der Karpule 13 einwirken, um die medizinische Substanz aus der Karpule 13 auszuschütten.

In der Figur 2 ist der Injektor 1 in einem Längsschnitt in einer Ausgangsstellung gezeigt. Zum Einstellen einer Dosis wird die Dosierhülse 30 an ihrem Griff 33 relativ zum Gehäuse 10 in der Einstellrichtung relativ zum Gehäuse 10 gedreht. Da die Dosierhülse 30 in Gewindeeingriff mit der inneren Gehäusehülse 20 steht, wird sie dadurch aus dem Gehäuseeinsatz 20 herausgeschraubt. Die auf der Dosierhülse 30 aufgedruckte Zahlenskala ist durch die Öffnungen 16 in der äusseren Gehäusehülse 14 ersichtlich und hilft beim Einstellen der gewünschten Dosis.

Die Kupplungshülse 50 ist über die Ratschenarme 53, welche mit ihren Nocken 54 in die Längsnuten 22 der inneren Gehäusehülse 20 eingreifen, an einer Rotation in Einstellrichtung blockiert. Da die Kupplungshülse 50 blockiert ist, ist auch die mit dieser rotativ verbundene Kolbenstange 70 blockiert.

Wird versehentlich eine zu hohe Dosis eingestellt, kann die Dosis korrigiert werden, indem die Dosierhülse 30 wieder zurück in das Gehäuse 10 geschraubt wird. Die Kupplungshülse 50 ist weiterhin rotativ relativ zum Gehäuse 10 gehalten, da die Kraft um die vorgespannten Ratschenarme 53 aus den Längsnuten 22 zu bewegen grösser ist, als die Reibkraft zwischen Dosierhülse 30 und Kupplungshülse 50. Folglich können sich beim Einstellen und Korrigieren einer Dosis, wenn die Dosierhülse 30 in Einstellrichtung oder in Gegenrichtung gedreht wird, die Kupplungshülse 50 und damit die Kolbenstange 70 nicht drehen.

Da die Dosierhülse 30 beim Einstellen und Korrigieren einer Dosis relativ zur Kupplungshülse 50 rotiert, gleiten die radialen Zähne 36 der Dosierhülse 30 über die Zähne 58 der Klickarme 57 der Kupplungshülse 50 und erzeugen dadurch ein Klickgeräusch und Vibrationen. Somit wird in Einstellrichtung (Erhöhung der Dosis) wie auch in Gegenrichtung (Reduzieren der Dosis) eine akustische wie auch taktile Rückmeldung für den Benutzer erzeugt.

Beim Herausdrehen der Dosierhülse 30 aus dem Gehäuseeinsatz 20 wie auch beim Einschrauben der Dosierhülse in den Gehäuseeinsatz 30 greifen die in axialen Zähne 59 der Kupplungshülse 50 nicht in die axialen Zähne 34 der Dosierhülse 30 ein, wodurch Dosierhülse 30 und Kupplungshülse 50 relativ zueinander rotiert werden können.

Die Stoppmutter 40 wird axial beweglich und rotationsfest in den axialen Nuten 32 der Dosierhülse 30 geführt. Sie wird bei einer Drehung der Dosierhülse 30 zusammen mit der Dosierhülse 30 gedreht, wodurch die Stoppmutter 40 auf dem Aussengewinde 52 der Kupplungshülse 50 in proximaler Richtung geschraubt wird. Beim Korrigieren bzw. Zurückdrehen der Dosierhülse 30 wird die Stoppmutter 40 entsprechend wieder in distale Richtung geschraubt.

Um eine eingestellte Dosis auszuschütten, drückt der Benutzer in distaler Richtung auf den Ausschüttknopf 60. Dadurch verschiebt sich die Kupplungshülse 50 relativ zur Dosierhülse 30 in distaler Richtung. Die axialen Zähne 59 der Kupplungshülse 50 werden durch diese Verschiebung in Eingriff mit den axialen Zähne 34 der Dosierhülse 30 gebracht, wodurch die Kupplungshülse 50 rotativ mit der Dosierhülse 30 gekoppelt wird.

Durch die distal wirkende Kraft wird die Dosierhülse 30 zurück in das Gehäuse 10 geschraubt. Das bedeutet, wenn die Vorspannkraft der Ratschenarme 53 überwunden wird und die Nocken 54 aus den Längsnuten 22 gleiten, kann die Kupplungshülse 50 relativ zum Gehäuse 10 rotiert werden. Da die Kupplungshülse 50 nun rotativ mit der Dosierhülse 30 gekoppelt ist und die Dosierhülse 30 in Gewindeverbindung mit der der inneren Gehäusehülse 20 steht, beginnt sich die Dosierhülse 30 unter der distal wirkenden Druckkraft mit einer Schraubbewegung in das Gehäuse 10 einzuschrauben. Die Kupplungshülse 50 wird durch die drehende Dosierhülse 30 ebenfalls relativ zum Gehäuse 10 gedreht. Dadurch wird auch die mit der Kupplungshülse 50 rotativ verbundene Kolbenstange 70 gedreht, welche sich dadurch in distaler Richtung in das Innengewinde 21 der inneren Gehäusehülse 20 schraubt. Somit wird der Flansch 73 am distalen Ende der Kolbenstange 70 axial relativ zum Gehäuse 10 verschoben und kann den in der Karpule 13 befindliche Stopfen (nicht dargestellt) in distaler Richtung verschieben. Infolge wird die medizinische Substanz aus der Karpule 13 ausgeschüttet.

Die flexiblen Ratschenarme 53 der Kupplungshülse 50 werden bei der Rotation der Kupplungshülse 50 über die Längsnuten 22 bewegt, wodurch ein Klickgeräusch und eine Vibration erzeugt wird. Bedingt durch den abgerundeten Bereich 55 können die Ratschenarme 53 nur in Ausschüttrichtung über die Längsnuten 22 bewegt werden. In Gegenrichtung stehen die Abschlussflächen 56 der Nocken 54 an den Flanken der Längsnuten 22 an und verhindern somit eine Drehung der Kupplungshülse 50. Dadurch kann die mit der Kupplungshülse 50 rotativ verbundene Kolbenstange 70 nur in Ausschüttrichtung bewegt werden.

Wie erwähnt, entsteht beim Ausschütten der Dosis durch die rotative Kopplung keine Relativbewegung zwischen Kupplungshülse 50 und Dosierhülse 30. Dadurch wird die Stoppmutter 40 mitgedreht, sie wird aber nicht relativ zur Kupplungshülse 50 verschoben. Das bedeutet, sie wird nicht auf dem Aussengewinde 52 in distaler oder proximaler Richtung bewegt. Somit wird die Stoppmutter 40 stets nur beim Einstellen oder Korrigieren relativ zur Kupplungshülse 50 und zur Dosierhülse 30 bewegt. Die Gewindesteigung und die Dimension der Stoppmutter 40 ist so ausgelegt, dass die Stoppmutter 40 am proximalen Ende des Aussengewindes 52 mit einem Anschlag an der Kupplungshülse 50 anstösst, wenn die maximale insbesondere die total ausschüttbare Dosis eingestellt wurde. Dadurch wird sichergestellt, dass der Benutzer zwar mehrmals eine Dosis einstellen und ausschütten kann, aber dass nicht eine Dosis eingestellt werden kann, welche die Kapazität der Karpule 13 oder eine andere Dosissumme überschreitet.

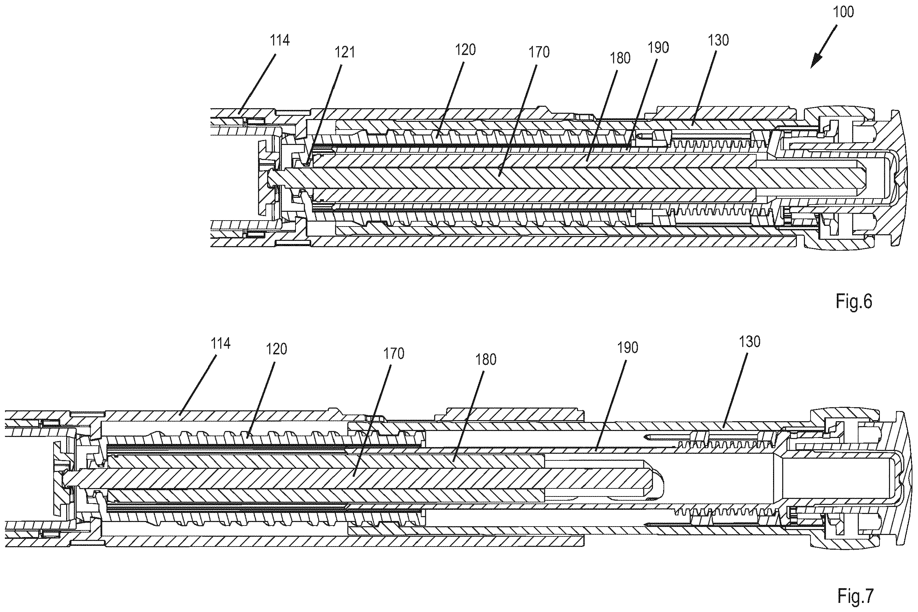

In den Figuren 6 und 7 ist eine weitere erfindungsgemässe Ausführung der Injektionsvorrichtung 100 mit einem Dosier- und Ausschüttmechanismus dargestellt, bei dem die Kupplungshülse zweiteilig ausgebildet ist. Im Unterscheid zur oben beschriebenen Ausführung umfasst die Kupplungshülse eine innere Kupplungshülse 180 und eine äussere Kupplungshülse 190. Die äussere Kupplungshülse 190 weist das oben beschriebene Gewinde für die Stoppmutter, die Klickarme, die Ratschenarme und die Zähne für die Kopplung mit der Dosierhülse 130 auf. Im Innern weist die äussere Kupplungshülse 190 axiale Nuten auf, in denen Stege (nicht dargestellt) der inneren Kupplungshülse 180 geführt sind, so dass die innere und äussere Kupplungshülse 180, 190 rotativ gekoppelt aber in Richtung Längsachse relativ zueinander verschiebbar sind.

Die innere Kupplungshülse 190 ist rotativ gekoppelt aber in Richtung Längsachse verschiebbar an der Kolbenstange 170 gelagert. Hierzu weist die innere Kupplungshülse 180 axiale Stege auf, welche wie oben beschrieben in axiale Nuten in der Kolbenstange 170 eingreifen. Zusätzlich kann die innere Kupplungshülse 180 mittels eines Flansches (nicht dargestellt) axial an der äusseren oder inneren Gehäusehülse 114, 121 gehalten sein, so dass sie relativ zum Gehäuse rotierbar ist.

Beim Einstellen einer Dosis wird die äussere Kupplungshülse 190 durch die Dosierhülse 130 in proximaler Richtung aus dem Gehäuse verschoben jedoch nicht rotiert, wie oben in Zusammenhang mit dem ersten Ausführungsbeispiel beschrieben. In Figur 7 ist die Injektionsvorrichtung 100 vor dem Ausschütten einer eingestellten Dosis dargestellt. Die innere und äussere Kupplungshülse 180, 190 werden beim Einstellen und Korrigieren einer Dosis nicht rotiert. Die innere Kupplungshülse 180 wird zudem axial nicht bewegt. Dadurch wird die äussere Kupplungshülse 190 relativ zur inneren Kupplungshülse 180 verschoben. Die beiden Kupplungshülsen 180, 190 bleiben dabei rotativ gekoppelt.

Beim Ausschütten einer eingestellten Dosis wird die äussere Kupplungshülse 190 mit der Dosierhülse 130 rotativ gekoppelt, wie beim ersten Ausführungsbeispiel erwähnt. Durch die nun rotierende äussere Kupplungshülse 190 rotiert auch die innere Kupplungshülse 180 und treibt somit die Kolbenstange 170 an. Diese schraubt sich durch das Innengewinde 121 in der inneren Gehäusehülse 120, wodurch der Flansch der Kolbenstange 170 nach distal bewegt wird und die medizinische Substanz aus der Karpule ausgeschüttet werden kann.

In einer weiteren Ausführung des erfindungsgemässen Dosier- und Ausschüttmechanismus kann das akustische und/oder taktile Signal beim Einstellen und Korrigieren einer Dosis anders realisiert sein als oben in der ersten Ausführung beschrieben. So kann beispielsweise anstelle der axialen Zähne 58 der Kupplungshülse und der axialen Zähne 34 der Dosierhülse eine Klickscheibe mit Zähnen und mit einem entsprechendem Flansch an Dosierhülse und Kupplungshülse vorgesehen werden wie sie in der ersten Ausführung der Patentanmeldung EP 19163197.7 auf den Seiten 15 - 24 beschreiben und in den Figuren 10 und 11 dargestellt ist. Ferner kann das akustische und/oder taktile Signal beim Einstellen und Korrigieren mittels einer ringförmigen Klickscheibe realisiert werden wie sie in der EP 19163197.7 auf Seite 24, Zeile 32 - Seite 25, Zeile 29 in Zusammenhang mit den Figuren 12 - 14 offenbart ist. Die Klickscheibe kann jedoch auch als Klickring gemäss den Figuren 15 - 17 und der entsprechenden Beschreibung auf Seite 25, Zeile 30 - Seite 27, Zeile 18 der EP 19163197.7 ausgebildet sein.

Weiter kann anstelle der Stoppmutter 40, welche sicherstellt, dass keine Dosis einstellbar ist, welche die Kapazität der Karpule überschreitet, ein anderer Begrenzungsmechanismus vorgesehen werden. So kann anstelle der Stoppmutter, des Aussengewindes 52 der Kupplungshülse, der Nuten 32 in der Dosierhülse der erfindungsgemässe Dosier- und Ausschüttmechanismus einen Begrenzungsmechanismus zwischen Dosierhülse 30 und Kupplungshülse 50 mit exzentrischen Elementen umfassen wie er in der Patentanmeldung EP 2 918 298 A1 in den Absätzen [0059 - 0138] und den Figuren 1 bis 25b beschrieben ist.

In einer weiteren Ausführung kann anstelle der Stoppmutter eine Kugel mit entsprechenden Führungsbahnen in der Kupplungshülse und in der Dosierhülse vorgesehen werden, wie in der Patentanmeldung WO 2010/149209 A1 auf den Seiten 25 - 27 beschrieben und in den Figuren 3 - 8 gezeigt. Alternativ kann anstelle der Kugel auch ein in Längsführungen geführtes Segment verwendet werden wie in der WO 2010/149209 A1 auf den Seiten 27 - 29 und den Figuren 9 - 14 offenbart.

Die Offenbarung der EP 19163197.7, der EP 2 918 298 A1 und der WO 2010/149209 A1 werden hiermit in die vorliegende Beschreibung miteinbezogen.

**BEZUGSZEICHENLISTE**

| | | | |
|---|---|---|---|
| 1, 100 | Injektor | 50 | Kupplungshülse |
| 10 | Gehäuse | 51 | Stege |
| 11 | Schutzkappe | 52 | Aussengewinde |
| 12, 120 | Karpulenhalter | 53 | Ratschenarme |
| 13 | Karpule | 54 | Nocken |
| 14, 114 | äussere Gehäusehülse | 55 | abgerundeter Bereich |
| 16 | Öffnung | 56 | Abschlussfläche |
| 17 | Rippen | 57 | Klickarme |
| | | 58 | Zahn |
| 20, 120 | innere Gehäusehülse | 59 | axiale Zähne |
| 21, 121 | Innengewinde | | |
| 22 | Längsnuten | 60 | Ausschüttknopf |
| 23 | Aussengewinde | 61 | Stege |
| 24 | distaler Steg | | |
| 25 | proximaler Steg | 70, 170 | Kolbenstange |
| | | 71 | Aussengewinde |
| 30, 130 | Dosierhülse | 72 | Nuten |
| 31 | Innengewinde | 73 | Flansch |
| 32 | Nuten | | |
| 33 | Griff | 180 | innere Kupplungshülse |
| 34 | axiale Zähne | 190 | äussere Kupplungshülse |
| 35 | Wulst | | |
| 36 | radiale Zähne | | |
| | | | |
| 40 | Stoppmutter | | |
| 41 | Stege | | |

## Patentansprüche

1. Eine Injektionsvorrichtung (1) zum Ausschütten einer Dosis eines Produkts mittels manuellem Vorschub einer Kolbenstange (70) in eine in der Injektionsvorrichtung (1) gehaltenen Karpule (13), mit einem Dosier- und Ausschüttmechanismus umfassend
ein Gehäuse (10) mit einer Längsachse;
eine im Gehäuse (10) gelagerte Dosierhülse (30) zum Einstellen und Korrigieren der Dosis;
einen Gehäuseeinsatz (20), welcher gehäusefest ist und innerhalb der Dosierhülse (30) angeordnet ist;
eine im Gehäuse (10) gelagerte Kupplungshülse (50) zum Antreiben der Kolbenstange (70),
wobei zum Einstellen und Korrigieren einer Dosis die Dosierhülse (30) relativ zur Kupplungshülse (50) drehbar ist und zum Ausschütten der Dosis die Dosierhülse (30) mit der Kupplungshülse (50) rotationsfest koppelbar ist,
**dadurch gekennzeichnet, dass**
die Kupplungshülse (50) ein Sperrelement aufweist, welches mit dem Gehäuseeinsatz (20) zusammenwirken kann, so dass die Kupplungshülse (50) in eine erste Drehrichtung rotierbar und in eine entgegengesetzte, zweite Drehrichtung nicht rotierbar ist relativ zum Gehäuseeinsatz (20).

2. Injektionsvorrichtung (1) nach Anspruch 1, **dadurch gekennzeichnet, dass** das Sperrelement ein elastischer radialer Arm (53) ist.

3. Injektionsvorrichtung (1) nach Anspruch 2, **dadurch gekennzeichnet, dass** der Arm (53) in einer Mantelfläche der Kupplungshülse (50) angeordnet ist.

4. Injektionsvorrichtung (1) nach einem der Ansprüche 1 bis 3, **dadurch gekennzeichnet, dass** der Gehäuseeinsatz (20) axiale Nuten (22) umfasst, mit denen das Sperrelement zusammenwirken kann.

5. Injektionsvorrichtung (1) nach einem der Ansprüche 1 bis 4, **dadurch gekennzeichnet, dass** der Gehäuseeinsatz (20) auf seiner Aussenseiten ein Gewinde (23) aufweist, mit dem die Dosierhülse (30) in Gewindeeingriff steht.

6. Injektionsvorrichtung (1) nach einem der Ansprüche 1 bis 5, **dadurch gekennzeichnet, dass** die Kupplungshülse (50) ein elastisches Klickelement (57) aufweist, welches beim Einstellen und Korrigieren einer Dosis in der ersten wie auch in der zweiten Drehrichtung mit der Dosierhülse (30) zusammenwirken kann zum Erzeugen eines akustischen und/oder taktilen Signals.

7. Injektionsvorrichtung (1) nach einem der Ansprüche 1 bis 6, **dadurch gekennzeichnet, dass** die Kupplungshülse (50) rotationsfest und verschiebbar relativ zur Kolbenstange (70) an dieser gelagert ist.

8. Injektionsvorrichtung (1) nach Anspruch 7, **dadurch gekennzeichnet, dass** die Kupplungshülse (50) auf ihrer Innenseite einen axialen Steg (51) aufweist und die Kolbenstange (70) eine axiale Nut (72) aufweist, in welche der Steg (51) eingreifen kann.

9. Injektionsvorrichtung (1) nach einem der Ansprüche 1 bis 8, **dadurch gekennzeichnet, dass** die Kupplungshülse einen erster Teil umfasst, welcher mit der Kolbenstange zusammenwirken kann und einen zweiter Teil umfasst, welcher mit der Dosierhülse koppelbar ist, wobei der erste und der zweite Teil relativ zueinander verschiebbar und rotationsfest gelagert sind.

10. Injektionsvorrichtung (1) nach Anspruch 9, **dadurch gekennzeichnet**, der erste Teil verschiebbar und rotationsfest mit der Kolbenstange relativ zur Kolbenstange an dieser gelagert ist und der zweite Teil hülsenförmig ausgebildet ist und den ersten Teil umgibt

11. Ein Dosier- und Ausschüttmechanismus für eine Injektionsvorrichtung (1) zum Ausschütten einer Dosis eines Produkts mittels Vorschub einer Kolbenstange (70), umfassend ein Gehäuse (10) mit einer Längsachse;
eine im Gehäuse (10) gelagerte Dosierhülse (30) zum Einstellen und Korrigieren der Dosis;
einen Gehäuseeinsatz (20), welcher gehäusefest ist und innerhalb der Dosierhülse (30) angeordnet ist;
eine im Gehäuse (10) gelagerte Kupplungshülse (50) zum Antreiben der Kolbenstange (70),
wobei zum Einstellen und Korrigieren einer Dosis die Dosierhülse (30) relativ zur Kupplungshülse (50) drehbar ist und zum Ausschütten der Dosis die Dosierhülse (30) mit der Kupplungshülse (50) rotationsfest koppelbar ist,
**dadurch gekennzeichnet, dass**
die Kupplungshülse (50) ein Sperrelement aufweist, welches mit dem Gehäuseeinsatz (20) zusammenwirken kann, so dass die Kupplungshülse (50) in eine erste Drehrichtung rotierbar und in eine zweite Drehrichtung nicht rotierbar ist relativ zum Gehäuseeinsatz (20).
